# EUROPEAN PATENT SPECIFICATION

(11) **EP 3 678 529 B1**
(45) Date of publication and mention of the grant of the patent: **13.07.2022**
(21) Application number: 18810174.5
(22) Date of filing: 01.06.2018
(51) Int. Cl.: A61B 1/00, A61B 5/097, A61M 15/08, A61M 16/06, A61M 16/08, A62B 18/02, A61M 16/10

(54) **OXYGEN MASK**
SAUERSTOFFMASKE
MASQUE À OXYGÈNE

(30) Priority: 02.06.2017 US 201762514770 P
(43) Date of publication of application: 15.07.2020
(73) Proprietor: Hussain, Shabina, Great Neck, New York 11020 (US); Alvi, Noerah, Great Neck, New York 11020 (US)
(72) Inventor: Hussain, Shabina, Great Neck, New York 11020 (US); Alvi, Noerah, Great Neck, New York 11020 (US)
(74) Representative: McQueen, Andrew Peter
(86) International application number: PCT/US2018/035681
(87) International publication number: WO 2018/223050

(56) References cited:
- EP-A2- 1 525 895
- US-A- 2 845 927
- US-A- 4 328 797
- US-A1- 2011 100 368
- US-A1- 2014 107 517
- US-A1- 2014 107 517
- US-A1- 2016 038 709
- US-A1- 2017 035 981

## Description

### BACKGROUND OF THE INVENTION

### 1. Field of the Invention

One or more embodiments of the invention relates generally to medical supplies. More particularly, the invention relates to an oxygen mask with capnometer and side port for use in sedated patients undergoing procedures requiring instrumentation of the mouth such as endoscopy or transesophageal echocardiography.

### 2. Description of Prior Art and Related Information

The following background information may present examples of specific aspects of the prior art (e.g., without limitation, approaches, facts, or common wisdom) that, while expected to be helpful to further educate the reader as to additional aspects of the prior art, is not to be construed as limiting the present invention, or any embodiments thereof, to anything stated or implied therein or inferred thereupon.

Oxygen delivery by nasal cannula allows access to the mouth / oral opening, such as insertion of various types of endoscopy probes. Patients undergoing an upper endoscopy or transesophageal echocardiography need to be sedated. Sedated patients require supplemental oxygen. Currently, supplemental oxygen is delivered using a nasal cannula. This method of oxygen delivery is inadequate for patients with compromised cardiopulmonary function, obesity and/or sleep apnea because the oxygen saturation drops, requiring probe withdrawal and subsequent rescue ventilation with a bag-valve (AMBU) mask. This can result in having to interrupt the sedation and/or the procedure.

Oxygen delivered by a non-rebreathing face mask is higher than a nasal cannula. However, the non-rebreathing face mask covers the mouth.

As can be seen, there is a need for an improved oxygen face mask that allows high concentration oxygen delivery in a sedated patient undergoing procedures requiring instrumentation of the mouth such as endoscopy or transesophageal echocardiography. Document US 2014/107517 discloses an oxygen face mask into which a side port can be cut to allow insertion of probes into patient's mouths while allowing delivery of a higher oxygen concentration.

### SUMMARY OF THE INVENTION

Embodiments of the present invention provide an improved oxygen face mask that allows high concentration oxygen delivery in a sedated patient undergoing procedures requiring instrumentation of the mouth such as endoscopy or transesophageal echocardiography. The invention is defined by the appended claims. Subject-matter referred as embodiments, disclosures and/ or inventions which are not claimed are not part of the invention.

The present invention provides a face mask comprising a dome-shaped mask body configured for placement over at least a portion of a nose and mouth of a patient; an elongated side of the mask body causing first side of the mask to extend longer than an opposite side of the mask, where an inferior aspect of the mask interconnects the first side of the mask to the second, opposite side of the mask; an oxygen port disposed through the mask body and positioned off-center toward the elongated side of the face mask; and a reinforcing rib disposed alongside the inferior aspect of the mask.

Another exemplary embodiment of the present invention provides a face mask comprising a dome-shaped mask body configured for placement over at least a portion of a nose and mouth of a patient; an elongated side of the mask body causing first side of the mask to extend longer than an opposite side of the mask, where an inferior aspect of the mask interconnects the first side of the mask to the second, opposite side of the mask; a reinforcing rib disposed alongside the inferior aspect of the mask; a keyhole notch formed in each side of the mask; and a keyhole cutout formed in each side of the mask, the keyhole cutout communicating with the keyhole notch.

Another exemplary embodiment of the present invention provides a face mask comprising a dome-shaped mask body configured for placement over at least a portion of a nose and mouth of a patient; an elongated side of the mask body causing first side of the mask to extend longer than an opposite side of the mask, where an inferior aspect of the mask interconnects the first side of the mask to the second, opposite side of the mask; a reinforcing rib disposed alongside the inferior aspect of the mask, wherein the inferior aspect curves in a concave manner from the first side to the opposite side; an oxygen port disposed through the mask body and positioned off-center toward the elongated side of the face mask; a reinforcing rib disposed alongside the inferior aspect of the mask; a keyhole notch formed in each side of the mask; and a keyhole cutout formed in each side of the mask, the keyhole cutout communicating with the keyhole notch.

In some embodiments, the face mask further includes a plurality of lateral ribs extending from an outer perimeter of the mask toward a central midline of the mask.

In some embodiments, the lateral ribs are positioned on both sides of the mask, sandwiching a keyhole cutout formed in each side of the mask.

In some embodiments, the face mask includes a T-shaped strap attachment pin extending from a surface of the mask on each side thereof.

In some embodiments, the attachment pin is disposed immediately toward an interior of the mask adjacent a keyhole cutout of the mask, the keyhole cutout operable to permit nasal cannula tubing to pass threrethrough.

These and other features, aspects and advantages of the present invention will become better understood with reference to the following drawings, description and claims.

### BRIEF DESCRIPTION OF THE DRAWINGS

Some embodiments of the present invention are illustrated as an example and are not limited by the figures of the accompanying drawings, in which like references may indicate similar elements.
FIG. 1 is a front view of a face mask according to an exemplary embodiment of the present invention;
FIG. 2 is a perspective view of a kit including the face mask of FIG. 1 with a nonrebreather bag and nasal cannula, according to an exemplary embodiment of the present invention;
FIG. 3 is a perspective view of the face mask of FIG. 1 in use on a patient;
FIG. 4 is a detailed side view of the nasal cannula attachment portion thereof;
FIG. 5 is a detailed top view of a nasal cannula in position in the nasal cannula attachment portion;
FIG. 6 is a detailed top view of the nasal cannula attachment portion and the T-hook for securing a strap, according to an exemplary embodiment of the present invention;
FIG. 7 is a side view of the face mask of FIG. 1; and
FIG. 8 is a back view of the face mask of FIG. 1.

Unless otherwise indicated illustrations in the figures are not necessarily drawn to scale.

The invention and its various embodiments can now be better understood by turning to the following detailed description wherein illustrated embodiments are described. It is to be expressly understood that the illustrated embodiments are set forth as examples and not by way of limitations on the invention as ultimately defined in the claims.

### DETAILED DESCRIPTION OF THE PREFERRED EMBODIMENTS AND BEST MODE OF INVENTION

The terminology used herein is for the purpose of describing particular embodiments only and is not intended to be limiting of the invention. As used herein, the term "and/or" includes any and all combinations of one or more of the associated listed items. As used herein, the singular forms "a," "an," and "the" are intended to include the plural forms as well as the singular forms, unless the context clearly indicates otherwise. It will be further understood that the terms "comprises" and/or "comprising," when used in this specification, specify the presence of stated features, steps, operations, elements, and/or components, but do not preclude the presence or addition of one or more other features, steps, operations, elements, components, and/or groups thereof.

Unless otherwise defined, all terms (including technical and scientific terms) used herein have the same meaning as commonly understood by one having ordinary skill in the art to which this invention belongs. It will be further understood that terms, such as those defined in commonly used dictionaries, should be interpreted as having a meaning that is consistent with their meaning in the context of the relevant art and the present disclosure and will not be interpreted in an idealized or overly formal sense unless expressly so defined herein.

In the following description, for purposes of explanation, numerous specific details are set forth in order to provide a thorough understanding of the present invention. It will be evident, however, to one skilled in the art that the present invention may be practiced without these specific details.

The present disclosure is to be considered as an exemplification of the invention and is not intended to limit the invention to the specific embodiments illustrated by the figures or description below.

As is well known to those skilled in the art, many careful considerations and compromises typically must be made when designing for the optimal configuration of a commercial implementation of any system, and in particular, the embodiments of the present invention. A commercial implementation in accordance with the teachings of the present invention may be configured according to the needs of the particular application, whereby any aspect(s), feature(s), function(s), result(s), component(s), approach(es), or step(s) of the teachings related to any described embodiment of the present invention may be suitably omitted, included, adapted, mixed and matched, or improved and/or optimized by those skilled in the art, using their average skills and known techniques, to achieve the desired implementation that addresses the needs of the particular application.

Broadly, embodiments of the present invention provide an oxygen face mask with capnometer and side port for use in sedated patients undergoing endoscopy or transesophageal echocardiography. The mask can include an elliptical insertion port to maximize access to the oral opening for performing procedures as well as making it easier for awake patients who require an oxygen mask to eat and drink. A reinforcement elliptical band can tent the mask up to enhance oral access. A set of keyhole notches can keep the nasal cannula out of eye, secures the mask to the face and allows for easy partial/full removal of mask with tactile feedback. An oxygen inflow port may be located laterally, away from the oral opening. The mask can include a T-hook design that provides easy removal and reapplication when one side of patient's face is turned away from the anesthesiologist. The mask can include carbon dioxide sampling ports at the center of fenestration that allow for CO₂ monitoring from left or right. The mask may be rotated 180 degrees on the face to allow access to the nares. Thus, the mask can also be used during bronchoscopies through the nares.

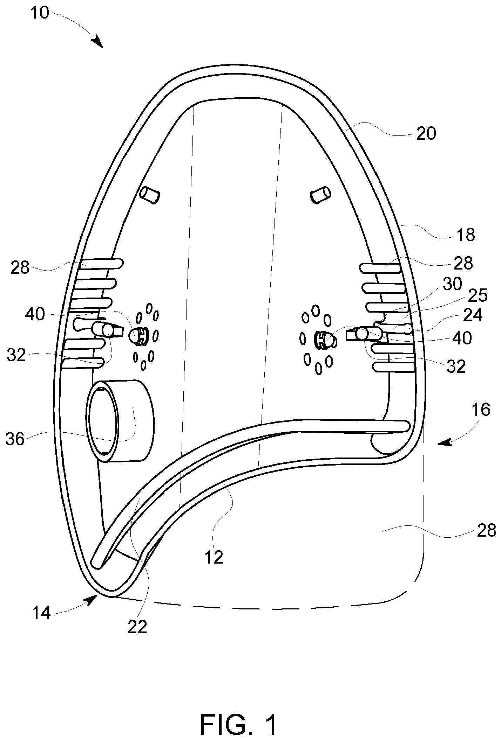

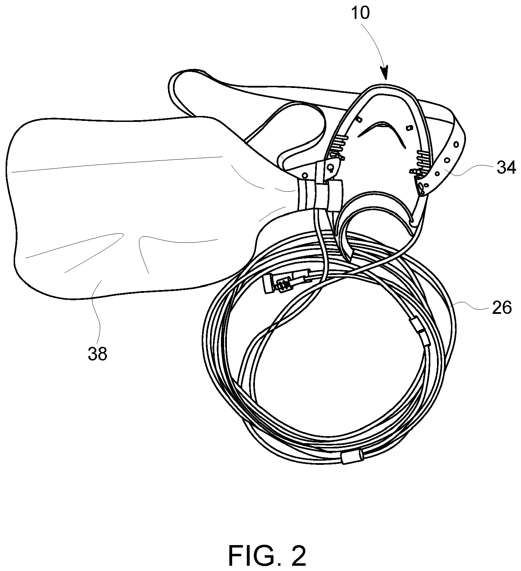

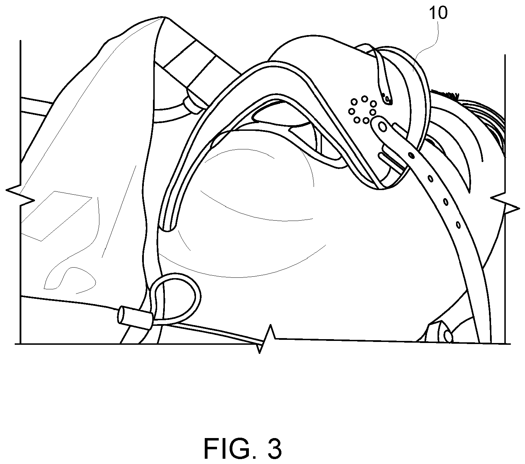

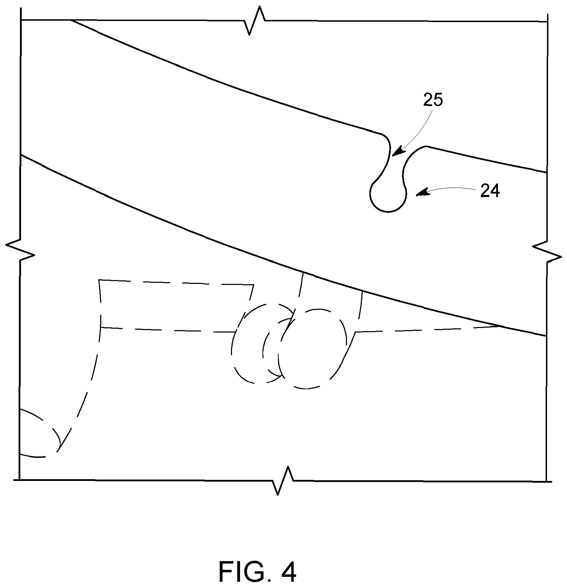

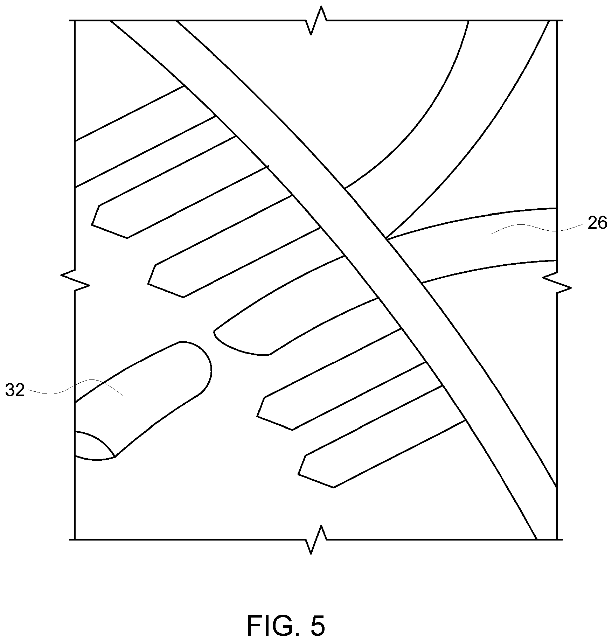

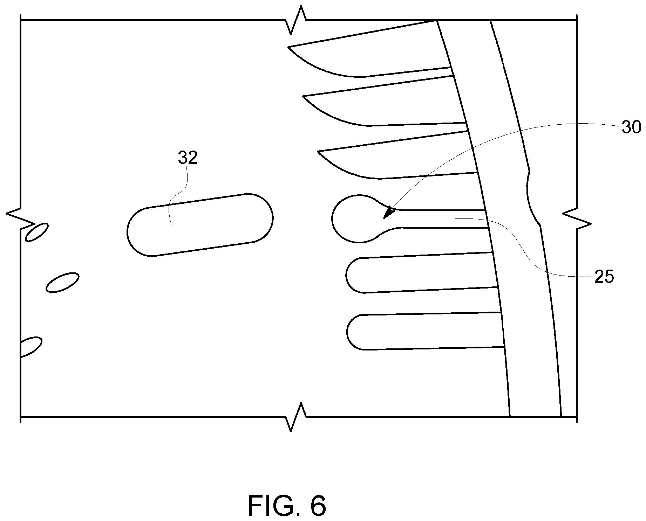

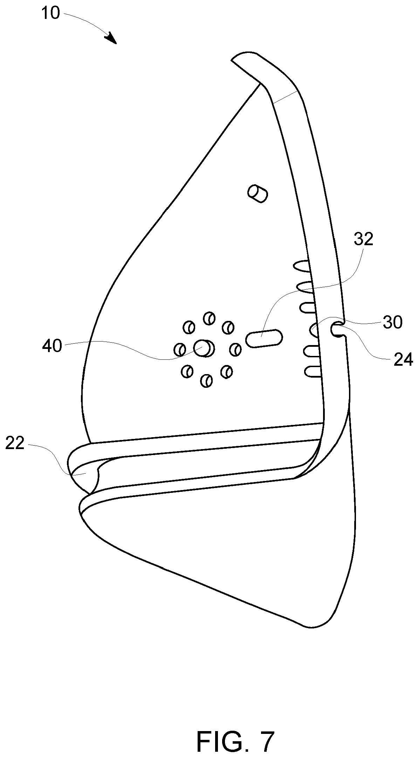

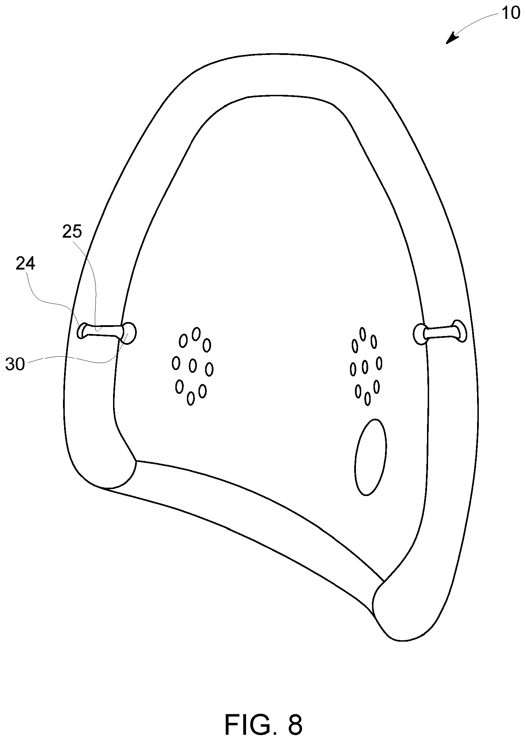

Referring to Figures 1 through 8, a mask 10, in some embodiments of the present invention, may be formed in the shape of an oval. In other embodiments, the mask 10 may closely resemble a round shape, egg-shaped, or the like. An inferior aspect 12 of the mask 10 may be interrupted by elliptical cutout 28, creating the mask 10 shown in FIG. 1. The resulting mask 10 includes an elongated side 14 extending alongside one side of the chin of the patient, as shown in FIG. 3, and a shortened side 16 that may terminate closer to the lips of the patient. The dotted line of FIG. 1 illustrates the shape of a conventional oxygen face mask. Of course, the cut-out may be formed on other shapes resembling the elliptical cutout as shown. This enables maximum access to the left side of the mouth during procedures. A perimeter 18 of the mask 10 may be reinforced with a continuous ridge 20, typically formed of plastic and typically having a stiffness greater than that of the body of the mask 10 itself. An elliptical insertion port 28, also referred to the elliptical cutout 28, can maximize access to the oral opening for performing procedures as well as allowing awake patients to eat and drink with the mask in place.

A rigid plastic rib 22 may be disposed along the inferior aspect 12 of the mask 10, generally parallel to the elliptical cut out, to provide rigidity and prevent the inferior aspect 12 of the mask 10 from excessive splaying onto the face when it is in use. The rib 22 may be formed with a stiffness greater than that of the body of the mask 10 to help maintain the shape of the mask 10 during use and enhance oral access.

Keyhole shaped notches 24 on both sides of the mask 10 help keep the nasal cannula tubing 26 secure where the tubing 26 exits the mask. Lateral ribs 28 above and below the notches 24 provide tactile feedback when snapping the nasal cannula tubing 26 in place and when separating the mask 10 from the nasal cannula. The ribs 28 also provide additional reinforcement for a keyhole cutout 30 integral with the key hole shaped notches 24.

A narrowed region 25 of the keyhole shaped notches 24 can terminate at an expanded diameter end 30 along the surface of the mask 10. Thus, the keyhole shaped notches 24, narrowed region 25, and expanded diameter end 30 can form a double keyhole shape. This design may allow for removal of the mask 10 while keeping the nasal cannula on the patient. The tubing 26 of the nasal cannula may extend from the patient's nostril, under the mask 10, to exit the mask at the expanded diameter end 30, travel along the exterior of the mask along the narrowed region 25 and pass back inside the mask to exit through the keyhole shaped notch 24. Thus, to remove the mask, the tubing 26 may simply be forced through the narrowed region 25 and out of the notch 24. Further, the keyhole notches 24 keeps the nasal cannula tubing 26 out of the eyes of the patient, secures the mask 10 to the patient's face and allows for easy partial/full removal of mask 10 with tactile feedback.

Two rigid or semi-rigid T-hooks 32 can provide a means to secure the removable mask strap 34. The flexible strap 34 can include holes in it and is stretched and placed over the T-hooks 32. The T-hook 32 may be strategically aligned with the keyhole cutout 30 to provide the strap 34 with a path that is aligned with the nasal cannula tubing 26. This keeps the nasal cannula snugly in place as the strap 34 is engaged. The inline location of the T-hook 32 secures the mask 10 to the patient's face and stabilizes the nasal cannula tubing 26 in place. The T-hook design provides for easy removal and reapplication when one side of patient's face is turned away from the anesthesiologist. While T-hooks 32 are shown in the Figures, other attachment pins may be contemplated within the scope of the present invention. For example, the T-hooks may be replaced by L-hooks, J-hooks, straight or curved pins, or the like.

An oxygen inflow port 36 is typically located on the left side of the mask 10, or on the side of the mask having the elongated side 14 (which is the left side in FIG. 1, but this configuration may be reversed, if desired, within the scope of the present invention). The oxygen inflow port 36 is designed to accommodate a reservoir bag 38, self-inflating bag or a variety of oxygen inflow systems with or without an adaptor. The lateral location of the oxygen inflow port 36 places the rebreather bag away from the oral opening. The universal port permits choice of oxygen inflow. In some embodiments, a self-inflating reservoir bag (AMBU) may be used at the universal port of the mask to provide positive pressure ventilation of a patient.

A bilateral carbon dioxide sampling port 40 may be disposed at the center of fenestration. This allows for CO₂ monitoring from the left side or the right side.

The mask may be rotated 180 degrees on the face to allow access to the nares. Thus, the mask can also be used during bronchoscopies through the nares.

The mask, as herein described, is the mask of choice for several applications. For example, the mask of the present invention may be especially useful for patients undergoing procedures under sedation requiring oral instrumentation, such as upper endoscopies, transesophageal echocardiography (TEE), bronchoscopies, and the like. The mask of the present invention may also be especially useful in the ICU, particularly post extubation, in ambulances, in hospitals, in hospice care, and the like.

All the features disclosed in this specification, including any accompanying abstract and drawings, may be replaced by alternative features serving the same, equivalent or similar purpose, unless expressly stated otherwise. Thus, unless expressly stated otherwise, each feature disclosed is one example only of a generic series of equivalent or similar features.

Claim elements and steps herein may have been numbered and/or lettered solely as an aid in readability and understanding. Any such numbering and lettering in itself is not intended to and should not be taken to indicate the ordering of elements and/or steps in the claims.

Many alterations and modifications may be made by those having ordinary skill in the art without departing from the scope of the invention.

## Claims

1. A face mask (10) comprising:
a dome-shaped mask body configured for placement over at least a portion of a nose and mouth of a patient;
an elongated side (14) of the mask body extends longer than a shortened side (16) of the mask, the elongated side (14) extending alongside one side of a chin of the patient when the face mask is worn, the shortened side (16) terminating adjacent lips of the patient when the face mask is worn, the shortened side (16) and the elongated side (14) defining left and right hand sides of the face mask when worn, where a bottom portion (12) of the mask interconnects the elongated side (14) of the mask to the shortened side (16) of the mask, **characterized in that**:
an oxygen port (36), disposed through the mask body, is positioned off-center toward the elongated side (14) of the face mask; and
a reinforcing rib (22) is disposed alongside the bottom portion (12) of the mask, reinforcing rib (22) having a stiffness greater than that of the mask body to help maintain a shape of the face mask during use and prevent the bottom portion (12) of the face mask from excessive splaying onto a face of the patient when in use.

2. The face mask of claim 1, wherein the bottom portion (12) curves in a concave manner from the elongated side (14) to the shortened side (16).

3. The face mask of claim 1, further comprising a plurality of lateral ribs (28) extending from an outer perimeter of the mask toward a central midline of the mask.

4. The face mask of claim 3, wherein the lateral ribs (28) are positioned on both sides of the mask, sandwiching a keyhole cutout (30) formed in each side of the mask.

5. The face mask of claim 1, further comprising a keyhole notch (24) formed in each side of the mask.

6. The face mask of claim 5, further comprising an expanded diameter portion at a narrow diameter end of the keyhole notch (24), forming a double keyhole shape with the keyhole notch (24).

7. The face mask of claim 1, further comprising a perimeter ridge (20) formed along an outer perimeter (18) of the mask, the perimeter ridge (20) having a stiffness greater than the mask body.

8. The face mask of claim 1, further comprising at least one carbon dioxide sampling port (40) formed through the mask body.

9. An oxygen delivery system comprising:
the face mask of claim 1,
wherein the bottom portion (12) curves outwardly, away from the patient when in use, from the elongated side (14) to the shortened side (16); and
a nasal cannula having tubing (26) removably secured to the face mask (10).

10. The system of claim 9, further comprising a plurality of lateral ribs (28) extending from an outer perimeter of the mask (10) toward a central midline of the mask (10).

11. The system of claim 9, wherein the face mask (10) includes a double keyhole cutout permitting the removable attachment of the nasal cannula tubing (26) to the face mask (10).

12. The face mask of claim 1 or the system of claim 9, further comprising a strap attachment pin extending from a surface of the mask (10) on each side thereof.

13. The face mask or system of claim 12, wherein the attachment pin is a T-shaped hook (32).

14. The face mask or system of claim 12, wherein the attachment pin is disposed immediately toward an interior of the mask adjacent a keyhole cutout (30) of the mask (10), the keyhole cutout (30) operable to permit nasal cannula tubing (26) to pass therethrough.

## Patentansprüche

1. Gesichtsmaske (10), umfassend:
einen kuppelförmigen Maskenkörper, der so gestaltet ist, dass er über mindestens einen Teil von Nase und Mund eines Patienten gelegt werden kann;
eine längliche Seite (14) des Maskenkörpers erstreckt sich länger als eine verkürzte Seite (16) der Maske, wobei sich die längliche Seite (14) entlang einer Seite des Kinns des Patienten erstreckt, wenn die Gesichtsmaske getragen wird. Die verkürzte Seite (16) endet neben den Lippen des Patienten, wenn die Gesichtsmaske getragen wird. Die verkürzte Seite (16) und die längliche Seite (14) definieren die linke und rechte Seite der Gesichtsmaske, wenn sie getragen wird, wobei ein unterer Abschnitt (12) der Maske die längliche Seite (14) der Maske mit der verkürzten Seite (16) der Maske verbindet, **dadurch gekennzeichnet, dass**:
eine Sauerstofföffnung (36), die durch den Maskenkörper hindurch angeordnet ist, außermittig in Richtung der länglichen Seite (14) der Gesichtsmaske positioniert ist; und
eine Verstärkungsrippe (22) entlang des unteren Abschnitts (12) der Maske angeordnet ist, wobei die Verstärkungsrippe (22) eine größere Steifigkeit als die des Maskenkörpers aufweist, um dazu beizutragen, eine Form der Gesichtsmaske während der Verwendung beizubehalten und zu verhindern, dass sich der untere Abschnitt (12) der Gesichtsmaske während der Verwendung übermäßig auf das Gesicht des Patienten ausdehnt.

2. Gesichtsmaske nach Anspruch 1, wobei der untere Teil (12) von der verlängerten Seite (14) zur verkürzten Seite (16) konkav gebogen ist.

3. Gesichtsmaske nach Anspruch 1, die ferner mehrere seitliche Rippen (28) aufweist, die sich von einem äußeren Umfang der Maske in Richtung einer zentralen Mittellinie der Maske erstrecken.

4. Gesichtsmaske nach Anspruch 3, wobei die seitlichen Rippen (28) auf beiden Seiten der Maske angeordnet sind und einen in jeder Seite der Maske ausgebildeten Schlüssellochausschnitt (30) umschließen.

5. Gesichtsmaske nach Anspruch 1, ferner mit einer Schlüssellochkerbe (24), die in jeder Seite der Maske ausgebildet ist.

6. Gesichtsmaske nach Anspruch 5, die ferner einen Abschnitt mit erweitertem Durchmesser an einem Ende mit schmalem Durchmesser der Schlüssellochkerbe (24) umfasst, der mit der Schlüssellochkerbe (24) eine doppelte Schlüssellochform bildet.

7. Gesichtsmaske nach Anspruch 1, ferner mit einer Umfangsrippe (20), die entlang eines äußeren Umfangs (18) der Maske ausgebildet ist, wobei die Umfangsrippe (20) eine größere Steifigkeit als der Maskenkörper aufweist.

8. Gesichtsmaske nach Anspruch 1, die ferner mindestens eine durch den Maskenkörper hindurch ausgebildete Kohlendioxid-Probenahmeöffnung (40) umfasst.

9. Sauerstoffabgabesystem, umfassend:
die Gesichtsmaske nach Anspruch 1,
wobei sich der untere Teil (12) bei Gebrauch von der verlängerten Seite (14) zur verkürzten Seite (16) nach außen, weg vom Patienten, wölbt; und
eine Nasenkanüle mit einem Schlauch (26), der abnehmbar an der Gesichtsmaske (10) befestigt ist.

10. Das System nach Anspruch 9 umfasst ferner eine Vielzahl von seitlichen Rippen (28), die sich von einem äußeren Umfang der Maske (10) zu einer zentralen Mittellinie der Maske (10) erstrecken.

11. System nach Anspruch 9, wobei die Gesichtsmaske (10) einen doppelten Schlüssellochausschnitt aufweist, der die abnehmbare Befestigung des Nasenkanülenschlauchs (26) an der Gesichtsmaske (10) ermöglicht.

12. Die Gesichtsmaske nach Anspruch 1 oder das System nach Anspruch 9 umfasst ferner einen Bandbefestigungsstift, der sich von der Oberfläche der Maske (10) auf jeder Seite derselben erstreckt.

13. Die Gesichtsmaske oder das System nach Anspruch 12, wobei der Befestigungsstift ein T-förmiger Haken (32) ist.

14. Gesichtsmaske oder System nach Anspruch 12, wobei der Befestigungsstift unmittelbar zum Inneren der Maske neben einem Schlüssellochausschnitt (30) der Maske (10) angeordnet ist, wobei der Schlüssellochausschnitt (30) so betätigt werden kann, dass ein Nasenkanülenschlauch (26) durch ihn hindurchgeführt werden kann.

## Revendications

1. Masque facial (10) comprenant :
un corps de masque en forme de dôme conçu pour être placé sur au moins une portion du nez et de la bouche d'un patient ;
un côté allongé (14) du corps de masque s'étend plus qu'un côté raccourci (16) du masque, le côté allongé (14) s'étendant le long d'un côté du menton du patient quand le masque facial est porté, le côté raccourci (16) se terminant à proximité des lèvres du patient quand le masque facial est porté, le côté raccourci (16) et le côté allongé (14) définissant des côtés gauche et droit du masque facial quand il est porté, une portion inférieure (12) du masque raccordant le côté allongé (14) du masque au côté raccourci (16) du masque, **caractérisé en ce que** :
un orifice d'oxygène (36), disposé à travers le corps de masque, est positionné de manière décentrée vers le côté allongé (14) du masque facial ; et
une nervure de renfort (22) est disposée le long de la portion inférieure (12) du masque, la nervure de renfort (22) ayant une rigidité supérieure à celle du corps de masque pour aider à maintenir la forme du masque pendant l'utilisation et empêcher la portion inférieure (12) du masque facial de trop s'écarter sur le visage du patient pendant l'utilisation.

2. Masque facial selon la revendication 1, dans lequel la portion inférieure (12) se courbe de manière concave depuis le côté allongé (14) vers le côté raccourci (16).

3. Masque facial selon la revendication 1, comprenant en outre une pluralité de nervures latérales (28) s'étendant depuis un périmètre externe du masque vers une ligne médiane centrale du masque.

4. Masque facial selon la revendication 3, dans lequel les nervures latérales (28) sont positionnées des deux côtés du masque, prenant en sandwich une découpe en trou de serrure (30) formée de chaque côté du masque.

5. Masque facial selon la revendication 1, comprenant en outre une encoche en trou de serrure (24) formée de chaque côté du masque.

6. Masque facial selon la revendication 5, comprenant en outre une portion à diamètre augmenté au niveau d'une extrémité à diamètre étroit de l'encoche en trou de serrure (24), constituant une double forme de trou de serrure avec l'encoche en trou de serrure (24).

7. Masque facial selon la revendication 1, comprenant en outre une arête périphérique (20) formée le long d'un périmètre externe (18) du masque, l'arête périphérique (20) ayant une rigidité supérieure à celle du corps de masque.

8. Masque facial selon la revendication 1, comprenant en outre au moins un orifice d'échantillonnage de dioxyde de carbone (40) formé à travers le corps de masque.

9. Système d'administration d'oxygène comprenant :
le masque facial selon la revendication 1,
dans lequel la portion inférieure (1) se courbe vers l'extérieur, en s'éloignant du patient lorsqu'il est utilisé, depuis le côté allongé (14) vers le côté raccourci (16) ; et
une canule nasale dotée d'une tubulure (26) fixée de manière amovible au masque facial (10).

10. Système selon la revendication 9, comprenant en outre une pluralité de nervures latérales (28) s'étendant depuis un périmètre externe du masque (10) vers une ligne médiane centrale du masque (10).

11. Système selon la revendication 9, dans lequel le masque facial (10) comprend une double découpe en trou de serrure permettant la fixation amovible de la tubulure de canule nasale (26) au masque facial (10).

12. Masque facial selon la revendication 1 ou système selon la revendication 9, comprenant en outre une broche de fixation par sangle s'étendant depuis une surface du masque (10) de chaque côté de celui-ci.

13. Masque facial ou système selon la revendication 12, dans lequel la broche de fixation est un crochet en forme de T (32).

14. Masque facial ou système selon la revendication 12, dans lequel la broche de fixation est placée immédiatement vers l'intérieur du masque à proximité d'une découpe en trou de serrure (30) du masque (10), la découpe en trou de serrure (30) pouvant être utilisée pour permettre la traversée de la tubulure de canule nasale (26).
